# EUROPEAN PATENT APPLICATION

(11) **EP 3 266 517 A1**
(43) Date of publication of application: **10.01.2018**
(21) Application number: 16178297.4
(22) Date of filing: 07.07.2016
(51) Int. Cl.: B01J 2/16, B01J 2/18, B07B 7/02, B08B 7/02

(54) **GRANULATOR FOR LIQUID SUBSTANCES, PARTICULARLY FOR UREA**

(71) Applicant: Casale SA, 6900 Lugano (CH)
(72) Inventor: RIZZI, Enrico, 22070 Casnate con Bernate (CO) (IT)
(74) Representative: Zardi, Marco

(57) **Abstract**

Granulator (1), in particular for urea, comprising an active system for removal of encrustations from at least one wall (4) with a surface (14) exposed to the granulation process, wherein said system operates by imparting a pulsed deformation to said wall.

## Description

### Field of application

The invention relates to the field of granulation processes, for example for the production of urea in solid form.

### Prior art

Granulation is a known technique for converting a liquid product into a solid form, consisting of essentially spherical granules of the desired size.

Granulation essentially consists in a gradual growth of the granules onto which the liquid feed is sprayed, the whole process being carried out within a dedicated granulator. The liquid forms a thin layer around the granule; said thin liquid layer rapidly solidifies under the effect of cooling air, thus increasing the size of the granule itself. In some cases, the granules are kept in a fluid bed condition, for example using air. In some cases, the process is also fed with a solid fraction which forms the "seeds" for initiating granulation.

One of the areas of application of the granulation process is the production of urea in solid form. Granulation is one of the main techniques for the production of solid urea together with prilling which, instead, involves cooling droplets of liquid urea falling inside a tower with countercurrent air.

Hence, granulation of urea is described in the relevant literature, for example in Meessen, "Urea", Ullmann's Encyclopedia of Industrial Chemistry, Wiley-VCH Verlag, 2010.

A urea granulator typically comprises a longitudinal granulation tank or chamber, essentially defined by a bottom and by side walls. Said walls are made of stainless steel sheets of suitable thickness, i.e. generally of about 3 mm. Inside the chamber the urea granules are kept in a fluid bed state by means of a suitable fluidizing air flow supplied from the bottom; the fluid bed has a component of longitudinal velocity which carries the material from an inlet section to an opposite outlet section and, moreover, may have a component of stirring movement, for example with a swirling action.

A process and apparatus for urea granulation of the type mentioned above is described in EP 1 707 258.

Granulation offers a series of recognized advantages. In the urea field, for example, granulation is considered to be superior to prilling because it provides a product with optimum characteristics in terms of mechanical strength, dimensional uniformity and surface finish. These characteristics allow the product to withstand storage and transportation in bulk form, without being damaged or without crumbling with dust generation and, consequently, they increase the commercial value of the product. Another advantage of granulation is the capability of producing large-size granules, for example with a diameter of about 3 mm or more, while the rival technique of prilling generally provides a diameter not greater than 2 mm.

The granulation technique, however, poses a number of technical problems. One of these problems consists in the encrustations inside the granulator. Solid formations, commonly called "crusts", may in fact be deposited on the walls of the granulator, owing to a local solidification of the growth liquid. Urea granulators are particularly prone to this problem since the urea easily tends to adhere to the walls of the granulator and to form crusts.

Said solid formations tend eventually to become detached and separate owing to their weight. This separation generally occurs when the solid formation has reached a mass such that the granulator no longer manages to expel it (i.e., for example, the fluid bed does not manage to convey it away); the crusts therefore accumulate on the bottom of the granulator where they may further increase in size owing to successive deposition and solidification of liquid and where they obstruct the flow cross-section of the apparatus and/or disturb the fluidizing air flow, thus altering the fluid dynamics of the process. This phenomenon is particularly damaging for fluid-bed granulators in which the internal fluid dynamics is essential for correct operation.

An attempt to reduce this drawback consists in using walls made of a material and/or having a degree of finish with a low adhesion factor. This measure, however, increases the costs and does not solve the problem because it has been noted that inside the granulator a very fine dust is generated, said dust being able to sediment even on surfaces with very high anti-adhesion properties, and the dust deposited on the walls acts as a substrate on which the crusts grow. It has been seen that the anti-adherent treatment of the surfaces reduces the size of the crusts before they separate, owing to the reduced adhesion factor of the surface, however it does not prevent the crusts from reaching dimensions such that they cannot be disposed of by the apparatus.

In general, the experience shows that the granulators must be periodically stopped to carry out internal cleaning and remove the encrustations, this resulting in stoppage of the plant or in any case loss of production. Moreover, the crusts formation depends on the conditions inside the granulator (geometry, temperature, etc.) and is a hardly predictable phenomenon. Therefore, it is difficult to establish with certainty the appropriate time intervals for cleaning which, by way of a precaution, must be carried out fairly frequently. In the field of urea granulators, for example, it has been seen that the cleaning operations should be carried at about monthly time intervals. This results in complications and additional costs and makes granulation less appealing despite the recognized excellent quality of the solid product.

### Summary of the invention

The invention aims to provide a new technique for avoiding or reducing in a significant manner the formation of encrustations on the walls of granulators and the consequent plant stoppages associated with the cleaning operations.

This object is achieved with a granulator according to claim 1. Said granulator comprises a system for removal of encrustations or dust from at least one wall of the granulation chamber, and said system comprises at least one shaking member which acts on said wall, imparting a pulsed deformation to said wall.

Said deformation preferably varies periodically, for example following a sinusoidal function of time.

Preferably said at least one shaking member is a mechanical vibrator. Said shaking member is for example an electric or pneumatic vibrator. For example, said shaking member is a piston vibrator or a cam vibrator.

In some embodiments of the invention the encrustation removal system acts on a wall with a double structure comprising a load-bearing wall and an internal wall facing the granulation chamber, and said encrustation removal system is arranged to impart said pulsed deformation to said internal wall.

In one embodiment, said internal wall bears against said load-bearing wall and is substantially in contact therewith. In another embodiment, said internal wall has its own support means and is spaced from said load-bearing wall.

Advantageously, the internal wall is a thin membrane wall, preferably made of thin metal sheet or of PTFE.

In some embodiments, said internal wall comprises a plurality of panels.

Preferably, the granulator comprises a plurality of shaking members acting in a distributed manner on the surface of said wall. Even more preferably, in the embodiments with double wall and internal panelled wall, at least one shaking member is provided for each panel of the internal wall. A further variant of the invention provides a single plate, said plate having a shaking member mounted thereon, which transmits the vibrations to several panels of the internal wall by means of lugs of said plate which bear against said panels.

The double-wall embodiments are also suitable for pneumatic type operation. In this case, the encrustation removal system introduces a pressurized fluid (for example air) between the load-bearing wall and the internal wall so as to deform the internal wall. Said shaking member therefore is, for example, a nozzle for introducing compressed air (or another pressurized fluid).

The internal wall is more easily deformable than the load-bearing wall and is subject to pressure-induced swelling (inflation effect); by varying the pressure of the fluid in a pulsed manner, a shaking effect of the internal wall is obtained which detaches possible encrustations.

It can be understood that the invention envisages the variants of: direct mechanical shaking, by means of at least one vibrating member in direct contact with the wall; indirect shaking by means of a pressurized fluid. Embodiments using both said shaking systems are also possible.

The granulator advantageously comprises a control system configured to activate said encrustation removal system manually or automatically at predefined intervals. Activation of the system according to the invention may be automatic, controlled by a control system. The control system preferably is of the distributed type, i.e. a distributed control system (DCS).

Another aspect of the invention relates to a method for removing encrustations from a wall of a granulator, according to the accompanying claims.

The basic principle of the invention is to induce an elastic deformation of the wall, which causes the breakage of the surface crust, entailing the detachment thereof. A fragile crust may be detached with a little deformation of the wall surface; a crust in a plastic state (for example owing to the high temperature) generally requires a greater deformation in order to be detached. An advantage of the shaking system is that, if activated at sufficiently short time intervals, it may also entail the removal of the dust just deposited, thus avoiding and preventing the formation of crusts.

The maximum distance of the deformed wall configuration from the undeformed wall configuration is called maximum deflection. The parameters such as the maximum deflection, the frequency and the duration of the pulses can be determined taking into account the ambient conditions inside the granulator and the nature of the crusts which must be removed. These parameters are also related to the characteristics of the internal wall, such as material, thickness and extension.

Advantageously, the maximum deformation imparted to said at least one wall is comprised between 0.5 mm and 1 mm. The frequency preferably ranges from 1 Hz to 20 Hz. The pulsations, even more advantageously, are activated discontinuously and cyclically. Even more advantageously, the pulsations have a time duration of between 1 and 5 seconds and are imparted at predetermined intervals. Said values of maximum deformation, frequency and duration of the pulsations are particularly preferred in the applications to a urea granulator.

High frequency and amplitude values are advantageously used when the temperature of the walls is high. As a general rule, the higher the temperature, the more the crusts become plastic and resistant to detachment and, consequently, require greater pulsation amplitude and frequency.

An advantage of the double-wall solution is the possibility of adopting different materials and design solutions for the two walls.

The load-bearing wall, which withstands the stresses, is not directly exposed to the environment of the granulation chamber and this is an advantage especially when the granulation chamber has an aggressive environment. The load-bearing wall consequently may be made of carbon steel, without using more costly materials such as, for example, the high-alloy steels which are used in urea plants for the parts which come into contact with the ammonium carbamate.

The internal wall is instead made of a material suitable for contact with the granulation environment, but must not withstand considerable mechanical stresses and therefore may have a thin wall thickness (membrane wall). In some applications of the invention said internal wall may also be made using a non-metallic synthetic material, for example PTFE (also known as Teflon). Said internal wall, basically, can be regarded as being a lining of the load-bearing wall.

The internal wall, when it is in contact with the external load-bearing wall, may be for example made of steel with a small thickness, preferably of less than 1 mm, more preferably less than 0.5 mm.

If the internal and external walls are separated by a space, the internal wall must withstand the pressure difference during operation (operative delta p) and may have for example a thickness of 2 to 3 mm.

Among the non-metallic materials, PTFE is particularly preferred not only because of its known non-adhesive properties, but also because of its elasticity, which allows the formation of an internal wall acting as a membrane, which reacts promptly to the pulsed pressure.

In some embodiments the walls of the granulator have a non-adhesive surface facing the granulation chamber. A non-adhesive surface limits further the deposition of encrustations and facilitates their separation under the action of the pulsed deformation of the wall. The non-adhesive surface can be obtained by means of suitable lining or painting of a sheet metal wall or, in the double wall embodiments, by making directly the internal wall from a material which is per se non-adhesive such as the already mentioned PTFE.

According to one of the aspects of the invention, the double-wall constructional design is such that the internal wall comprises a plurality of panels. Said panels, for example, are made of metal sheets or synthetic material.

Said panels may be applied directly onto said load-bearing wall in order to obtain a constructional design with coupled walls. In a preferred variant, said panels are applied directly onto the load-bearing wall and, moreover, each panel is sealed together with said load-bearing wall along a respective contour. An advantage of said embodiment is the better control of the deformation of the internal wall.

In spaced wall embodiments, i.e. when the walls are separated by a cavity, said panels are preferably welded along the contour onto a suitable support frame.

The granulator generally comprises a plurality of nozzles for spraying growth liquid. According to another aspect of the invention, said second wall has a greater deformability in the vicinity of said nozzles. This measure improves the cleaning in the zone around the nozzles, compensating for the fact that the nozzles constrain the internal wall limiting the movements thereof.

Said greater deformability may be obtained with wall portions having a smaller thickness around the nozzles or, in a further variant of the invention, using panels made of different material. For example, in one embodiment of the invention, sheet-metal panels with a regular form are used for the broad surfaces, and panels made of synthetic material are used in the particular zones subjected to greater constraint close to the nozzles.

In some embodiments of the invention the external wall and the internal wall define a substantially hermetically sealed chamber. In this case, in the pneumatic type embodiments, it is required to introduce pressurized air in a pulsed manner as well as to discharge the air between the pulses. This object can be achieved with suitable respective air inlet and air discharge valves controlled by a PLC.

The control system may operate by carrying out simultaneous cleaning of all the panels or, in other embodiments, sequential (rotational) cleaning of each single panel which forms the wall of the granulator. This mode of operation with sequential cleaning has the advantage of creating less disturbance to the granulator during operation and of using a nearly continuous, but limited, amount of power for cleaning.

The aforementioned sequential mode is particularly advantageous in the case of a pneumatic shaking system, where the simultaneous inflation of all the walls of the granulator would result in the use of very high instantaneous air flowrates, generally not available in the plant or in any case likely to cause perturbations to the rest of the plant. In this case, it is also possible to store pressurized air inside a dedicated tank so that the amount of air used is continuous and limited during filling of the tank, while during inflation of the walls high instantaneous flowrates are available without causing perturbations to the rest of the plant.

The invention is advantageously applied to one or more walls and/or to the bottom of a granulator. In a longitudinal granulator, the invention is preferably applied at least to the lateral longitudinal walls.

The main advantage of the invention consists in providing a system which actively acts against the deposition of encrustations. The system for cleaning and removing encrustations may in fact be activated on command, for example periodically, so as to keep the walls surfaces clean. Operation of the system is advantageously calibrated so as to remove the solid formations before they reach dimensions such as to interfere significantly with the process. The encrustations may be removed when they have a mass small enough to be transported by the fluid bed and, consequently, automatically expelled by the granulator. The encrustations may then be screened, separated from the granules and recycled if necessary.

The invention also allows remove the dust depositions before formation of actual crusts.

The system according to the invention may act during operation of the granulator without requiring any stoppage of the plant or access inside the granulator. The periodic activation of the system may in fact be managed by means of a suitable control system such as the already mentioned DCS.

In some embodiments the granulator may be controlled so that the cleaning system is differently activated to carry out cleaning for ordinary or extraordinary maintenance.

The cleaning system according to the invention is therefore an active system which allows better results to be obtained compared to the prior art techniques which substantially envisage only programmed maintenance and in any case require to stop the operation of the granulator.

The present invention therefore solves in a brilliant manner most of the problems associated with cleaning of a granulator. Any periodic cleaning operations (for example in zones which are particularly prone to encrustations, such as in the vicinity of the panel joints) may be carried out at longer time intervals and have a shorter duration compared to the current technology.

The invention is applicable preferably to urea granulators, but may be used advantageously also for granulators of other substances, for example ammonium nitrate.

The advantages of the invention will become even clearer with the aid of the following detailed description.

### Description of the figures

Fig. 1 is a schematic representation of a granulator, for example a urea granulator.
Fig. 2 shows a side wall of the granulator according to Fig.1, in a first embodiment of the invention.
Fig. 3 shows a variant of Fig. 2.
Fig. 4 shows a side wall of the granulator according to Fig.1, in a second embodiment of the invention.
Fig. 5 shows in schematic form a control system for the pneumatic type activation.

### Description of preferred embodiments

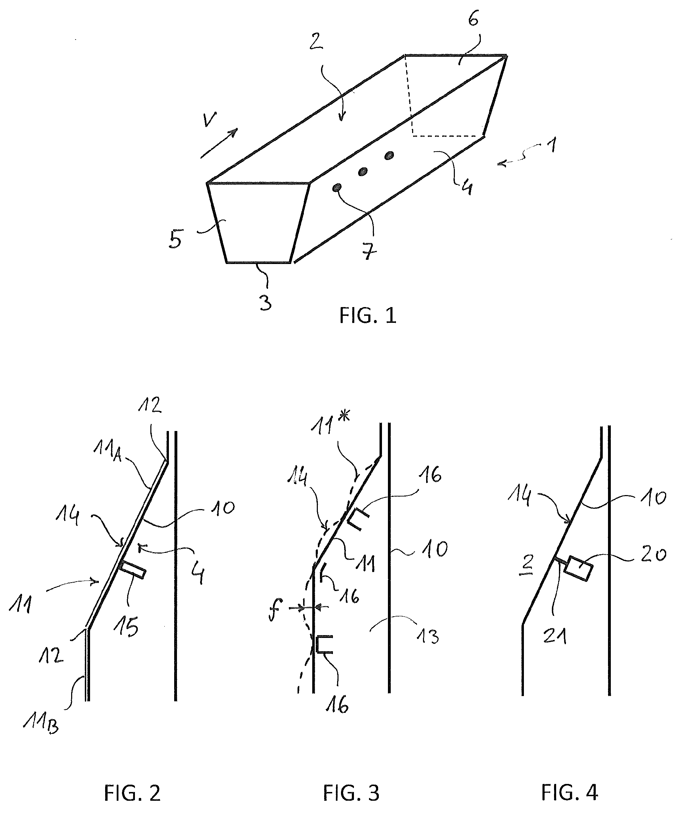

Fig. 1 shows in schematic form a urea granulator 1 which comprises a granulation chamber 2 defined by a bottom 3 and by side walls 4.

The granulator 1 has an essentially longitudinal crossing direction V from an inlet section 5 to an opposite outlet section 6. Inside the chamber 2, during operation, a fluid bed formed by the growing urea granules is created.

The granulator 1 comprises nozzles 7 which are distributed along the walls 2 and/or on the bottom 3. Said nozzles 7 spray liquid urea or highly concentrated urea solution (generally 96% or more) in order to feed the process.

Further details of the granulator 1 and the process are known to the person skilled in the art and do not need to be described for the purposes of understanding the invention.

Fig. 2 shows a schematic cross-section of a side wall 4 in a first embodiment of the invention.

Said wall 4 has a double structure comprising a load-bearing wall 10 and a membrane-type internal wall 11. The wall 11 is made for example of sheet metal with a small thickness (< 1 mm) or of synthetic material and is sealed together with the load-bearing wall 10 along a peripheral edge 12. Sealing along the edge 12 may be performed with a weld if the materials so permit, for example if both the walls 10 and 11 are made of steel.

The internal wall 11 has a surface 14 (opposite to the load-bearing wall 10) which faces the granulation chamber 2. In some embodiments said surface 14 has undergone an anti-adhesion treatment. The wall 11 is therefore the wall which actually defines the granulation chamber 2, while the load-bearing wall 10 has the structural function of withstanding the mechanical stresses.

More specifically, Fig. 2 shows a preferred embodiment in which the wall 11 comprises a plurality of panels; in the figures two panels 11_{A} and 11_{B} can be seen. The load-bearing wall 10 is thus lined with a panelling which, as a whole, forms the internal wall 11 facing the granulation chamber 2. Each panel 11_{A}, 11_{B}, etc., has preferably its own sealed edge 12.

The internal wall 11, in the embodiment shown in Fig. 2, substantially bears against the load-bearing wall 10. Said embodiment may therefore be referred to as being of the coupled-wall type. Compressed air may nevertheless be introduced between the two walls 10 and 11.

It should be noted that the internal wall 11 acts an internal lining for the load-bearing wall 10. Owing to the sealed edge 12, the load-bearing wall 10 is not directly exposed to the environment of the granulation chamber 2 and therefore does not need to be designed for direct contact with the urea. The materials of the walls 10 and 11 may therefore be of a varying nature.

Fig. 3 shows a variant with spaced walls. In this variant of the invention, the internal wall 11 is supported by a structure of beams or sections 16, so as to space from each other the external wall 10 and the internal wall 11, defining a cavity 13.

The panels of the internal wall 11 are welded to the load-bearing structure formed by the beams 16. In this way inflation of the internal wall is simultaneous for all the panels.

The internal wall 11, both in a coupled-wall embodiment as shown in Fig. 2 and in a spaced-wall embodiment as shown in Fig. 3, may have a greater deformability in the region of the nozzles 7 in order to compensate for the constraining effect of the nozzles themselves. Preferably, the wall 11 has a smaller thickness in a region around said nozzles 7 or is made of a more flexible material. For example, the wall 11 is made of PTFE in the zones around the nozzles 7, while the remaining parts of the same wall 11 are made of sheet metal.

The granulator comprises one or more shaking devices 15 which may be mechanical vibrators acting directly on the internal wall 11 (for example cam vibrators), or spouts arranged so as to introduce compressed air into the space between the load-bearing wall 10 and the internal wall 11.

The internal wall 11, under the action of the shaking members 15, is deformed and behaves substantially as a membrane. The dotted line 11* in Fig. 3 indicates the deformation of the internal wall 11, while the reference f in the same figure indicates the maximum deformation of said wall 11.

The deformation of the wall 11 follows the pulsed progression of the stress transmitted by the devices 15, with a shaking effect of the surface 14 which removes possible solid deposits (crusts) or urea dust.

One advantage of the division into panels 11_{A}, 11_{B}, ..., as shown in Fig. 2, where each of said panels has its own seal along the respective edge 12, consists in the fact that each panel has a small surface area and, consequently, ensures better control of the abovementioned deformation and swelling effect.

Preferably, a plurality of shaking panels 15 is provided, and even more preferably, at least one shaking device 15 is provided for each panel 11_{A}, 11_{B}, etc., of the internal wall 11.

Fig. 4 shows an embodiment in which the wall 4 has a single structure formed by the load-bearing wall 10 and the surface 14 consequently is formed by the inner side of said wall 10, directed towards the granulation chamber 2.

The pulsed deformation, in this single-wall embodiment, is imparted by means of direct contact, by one or more vibrators 20 which comprise at least one vibrating element 21 acting on the wall 10.

In the mechanical type embodiments (i.e. which use mechanical vibrators), the shaking members 15 are preferably components known per se. For example vibrators comprising a vibrating member, such as a piston, in direct contact with the wall of the granulator, or vibrators of the cam type, may be used. The latter comprise a casing which contains, mounted therein, a fast-rotating cam which, owing to its eccentricity, transmits vibrations to the casing. The casing of the cam vibrator, therefore, may be fixed directly to the wall of the granulator (for example the wall 11 of Figs. 2, 3 or the wall 10 of Fig. 4) or may be fixed to a plate provided with lugs (of the feeler type) which bear against the wall of the granulator.

In some embodiments of the invention, both the pneumatic and the mechanical systems acting on the same internal wall 11 are provided. Said two pneumatic and mechanical systems may be used, simultaneously or alternately, for example for periodic cleaning and for extraordinary cleaning, respectively.

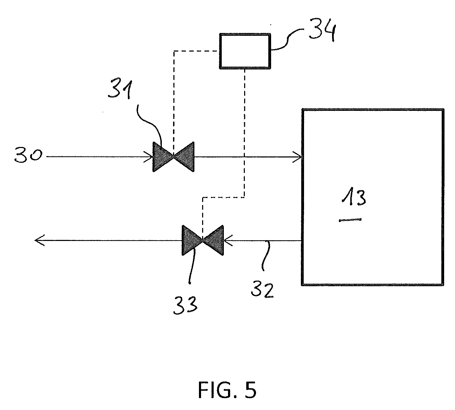

Fig. 5 shows in a simplified manner a system for introducing compressed air into the cavity 13 of Fig. 3, for embodiments of the pneumatic type. Said system essentially comprises an air inlet line 30, an air inlet valve 31, an air discharge line 32 with respective discharge valve 33, a PLC 34 which controls the valves 31 and 33.

In the rest condition, the system has the discharge valve 33 open and the inlet valve 31 closed. The control system 34 operates with a cleaning sequence which comprises the following steps:
the discharge valve 33 closes and the inlet valve 31 opens, allowing the compressed air supplied in the line 30 to flow into the cavity 13;
the pressure inside said cavity 13 consequently gradually increases, deforming the internal wall 11;
once a set pressure is reached, such as to produce a desired deformation of the internal wall, the PLC 34 closes the inlet valve 31 and opens the discharge valve 33.

Said sequence continues for the set operating time of the cleaning system, for example 5 seconds.

A system such as that shown in Fig, 5 may be used also to supply compressed air between the walls 10 and 11, in an coupled-wall system of the type shown in Fig. 2.

Pneumatically (and/or mechanically) imparted shaking of the wall of the granulator removes the encrustations or the dust (which, increasing, would form the crusts) from the surface 14 directly in contact with the fluid bed. The process for removing encrustations is performed with a suitable periodicity and may be manually or automatically controlled by a control system.

Based on the operating data, depending on the extent to which the crusts tend to form and depending on their consistency (more or less plastic or fragile), the activation interval of the cleaning sequence may be decided.

Frequent activation (e.g. every 5 minutes) may eliminate the dust as it is deposited, substantially avoiding the formation of crusts.

Less frequent activation may allow the formation of crusts and in this case the activation frequency is advantageously determined so that the crusts cannot reach the size and mass which would prevent their expulsion from the bed. In this way, the solid material which separates from the surface 14 may be transported by the fluid bed and expelled from the granulator 1, through the outlet section 6, instead of accumulating on the bottom 3.

The invention thus achieves the objects mentioned above, increasing the possibilities and prospects for using granulation for the production of solid urea, increasing the profitability owing to stoppages which are fewer and of shorter duration.

## Claims

1. Granulator (1) for obtaining a solid product in the form of granules, starting from a supply of said product in liquid form, said granulator comprising walls which define a granulation chamber (2), **characterized in that** it comprises a system for removing encrustations or dust from at least one wall (4), said system comprising at least one shaking member (15, 20) which acts on said wall (4) and which is configured to impart a pulsed deformation to said wall.

2. Granulator according to claim 1, **characterized in that** said at least one shaking member (15, 20) is a mechanical vibrator.

3. Granulator according to claim 1 or 2, **characterized in that**: said wall (4) has a double structure, comprising a load-bearing wall (10) and an internal wall (11) facing the granulation chamber (2) and said encrustation removal system is arranged to impart said pulsed deformation to said internal wall (11).

4. Granulator according to claim 3, wherein said internal wall (11) bears against said load-bearing wall (10) and is substantially in contact therewith.

5. Granulator according to claim 3, wherein said internal wall (11) has its own support means (16) and is spaced from said load-bearing wall (10).

6. Granulator according to any one of claims 3 to 5, said internal wall (11) being a thin membrane wall, preferably made of thin metal sheet or made of PTFE.

7. Granulator according to any one of claims 3 to 6, said internal wall (11) comprising a plurality of panels (11_{A}, 11_{B}).

8. Granulator according to any one of the preceding claims, **characterized in that** it comprises a plurality of shaking members acting in distributed manner on the surface of said wall (4).

9. Granulator according to claims 7 and 8, wherein said encrustation removal system comprises at least one shaking member for each panel (11_{A}, 11_{B}) or group of panels, of the internal wall (11).

10. Granulator according to any one of claims 3 to 9, wherein said at least one shaking member is adapted to introduce a pressurized fluid means between the load-bearing wall (10) and the internal wall (11) so as to deform the internal wall (11).

11. Granulator according to any one of the preceding claims, wherein the granulator comprises a plurality of nozzles (7) for spraying the growth liquid and said internal wall (4) has increased deformability in the vicinity of said nozzles (7).

12. Granulator according to any one of the preceding claims, wherein said wall (4) has a non-adhesive coating or lining (14) directed towards the granulation chamber.

13. Granulator according to any one of the preceding claims, **characterized in that** it operates in a fluid bed condition inside said granulation chamber (2).

14. Granulator according to any one of the preceding claims, comprising a control system configured to activate said encrustation removal system manually or automatically at predefined intervals.

15. Method for removing encrustations from a wall (4, 11) of a granulator, in particular of a urea granulator, said wall having a surface (14) exposed to a granulation chamber (2), the method comprising the operation of imparting a pulsed deformation to said wall, through direct mechanical contact with at least one vibrating member and/or through a pressurized fluid means.

16. Method according to claim 15, wherein the pulsed deformation has a frequency of between 1 Hz and 20 Hz.
